# EUROPEAN PATENT APPLICATION

(11) **EP 1 457 764 A1**
(43) Date of publication of application: **15.09.2004**
(21) Application number: 04001163.7
(22) Date of filing: 21.01.2004
(51) Int. Cl.: G01K 3/04

(54) **Device for indicating the residual life of industrial products**

(30) Priority: 12.03.2003 IT ge20030022
(71) Applicant: Thermochron S.r.l., 51100 Pistoia (IT)
(72) Inventor: Marbot, Isabelle M. J., 51100 Pistoia (IT)
(74) Representative: Porsia, Attilio, Dr.

(57) **Abstract**

Device for indicating the residual life of industrial products, comprising means (2) for sensing one or more environmental variables correlated with the preservation of the product, positioned on the interface between the product and the environment; processing means (1) for acquiring the data read by the sensor means (2), entering these data into a suitable program for evaluating the residual life of the product on the basis of these data, and returning the data relating to the residual life of the said product; and means (103, 203, 303) of displaying the data concerning the residual life of the product, controlled by the said processing unit (1).

## Description

The present invention relates to a device for indicating the residual life of industrial products, this device being particularly suitable for food products and pharmaceuticals.

Most industrial products, and particularly the products of the agricultural, food and pharmaceutical industries, have properties which decay until they are lost completely over a specific time interval, which is generally indicated in the form of the period of validity or expiry date of the product.

However, it should be noted that in general, as commonly indicated on the packages of the said products, this date relates to the unopened product stored in optimal conditions, although it is not always possible for the consumer to determine whether the product has been preserved in the most appropriate way before he purchases it.

US-A-5531180 discloses a device for determining the temperature variation which a product, particularly a frozen product, has undergone, and for indicating the extent of this variation by suitable means. However, this device does not provide any evaluation of the actual residual life of the product, and consequently the information which it obtains is difficult to transfer to the consumer, who does not usually have the technical knowledge or equipment for carrying out such evaluation.

The aim of the present invention is therefore to provide a device for providing the user with instant and simple information, not dependent on interpretation, concerning the state of preservation of a given product and its residual life.

The object of the present invention is therefore a device for indicating the residual life of industrial products, comprising means for sensing one or more environmental variables correlated with the preservation of the product, positioned on the interface between the product and the environment; processing means for acquiring the data read by the sensor means, entering these data into a suitable program for evaluating the residual life of the product on the basis of these data, and returning the data relating to the residual life of the said product; and means of displaying the data concerning the residual life of the product.

A further object of the present invention is a method for evaluating the residual life of a product, comprising the following steps: entering the characteristic parameters of a single type of product into a set of generic functions which represent the decay of the properties of a product as a function of time, a constant value of at least one environmental variable being assigned to each function; measuring, instant by instant, the values relating to the said environmental variable; entering data relating to the said values into the set of functions; determining the preservation history of the product and its residual life on the basis of the entered data; and displaying the residual life data determined in this way.

Further objects, characteristics and advantages of the present invention will be made clear by the following description of one of its embodiments, provided by way of example and without restrictive intent, and making reference to the attached drawings, in which:
Figure 1 is a schematic diagram representing an embodiment of the device according to the present invention;
Figure 2 is a graph illustrating the preservation history of a given product; and
Figures 3 to 6 are flow diagrams illustrating the operation of the device according to the present invention.

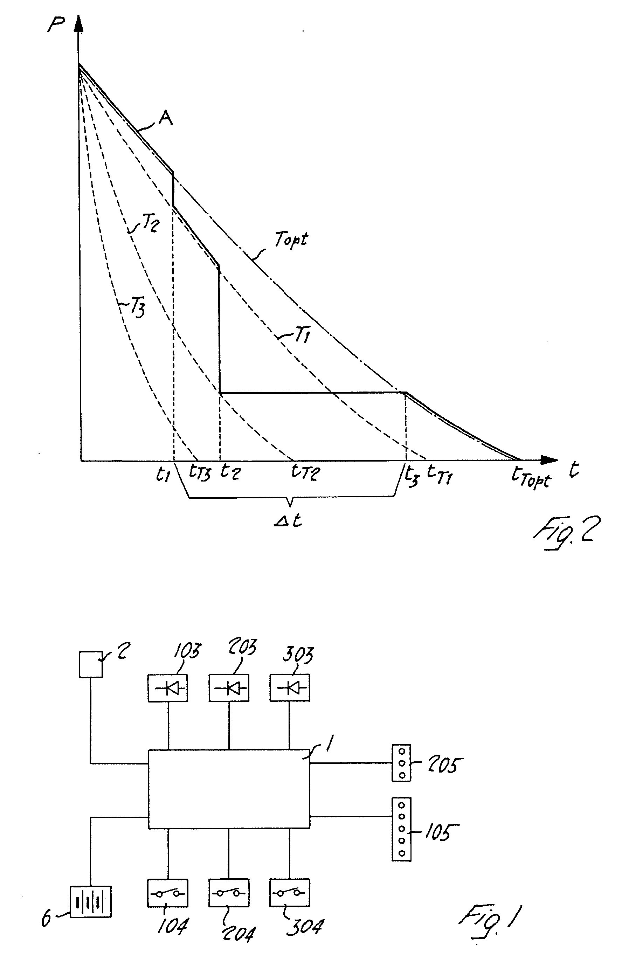

Figure 1 shows a diagram representing an embodiment of the device according to the present invention; the number 1 indicates the central processing unit. This processing unit is connected to a sensor 2 which measures an environmental variable, for example temperature, which can affect the decay of the properties of the product. The central unit 1 is also connected to three light-emitting diodes, these being green 103, yellow 203 and red 303, for indicating the state of preservation of the product by means of three different signals, the switches 104, 204 and 304 for entering parameters into the central unit 1 and resetting it, and the connectors 105 and 205 for entering data into the central unit 1; the circuit is supplied by the battery 6.

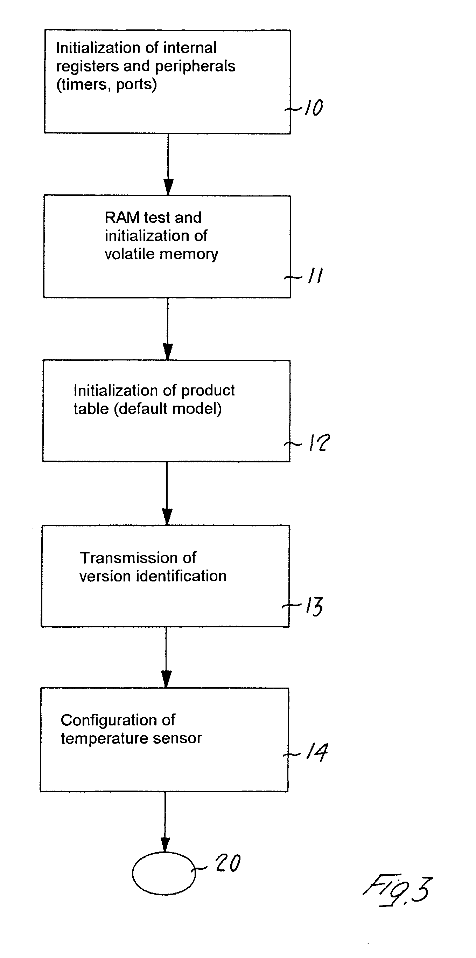

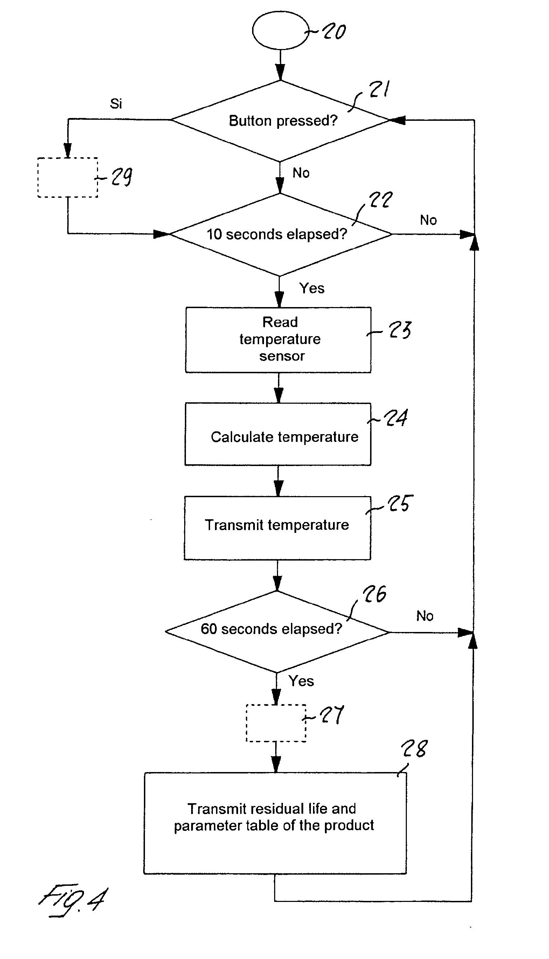

The operation of the device described above and the method according to the invention will be made clear by the following description which refers to Figures 2 to 6. In particular, Figure 3 shows a first flow diagram relating to the operation of the device of Figure 1; in the steps shown in this diagram, the appropriate initialization is first carried out (steps 10 and 12), as are the testing (11) and configuration (14) required for the operation of the device. At this level, apart from the system test (11), the most important operation is the initialization of the parameter table of the product (12), which for practical purposes makes the generic algorithm entered into the central processing unit 1 applicable to the specific case; this initialization is carried out in practice by interfacing the processing unit 1 of the device through its connectors 105 and 205 with suitable data entry means, for example a data output periphery of a computer which controls the preservation of the products.

The entered parameters will essentially consist of the maximum life of the product stored in optimal conditions and the relationship between the properties of the product and the environmental variable which is to be monitored, which in this specific case is the temperature. At this point, the central unit 1 can use these parameters to calculate for the product the set of curves shown schematically in Figure 2, which shows the relationship between the properties of the product, expressed as a percentage, and the preservation time; thus, for each preservation temperature, in other words the preservation variable observed by means of the sensor 2, a corresponding isotherm can be calculated, as shown in the figure.

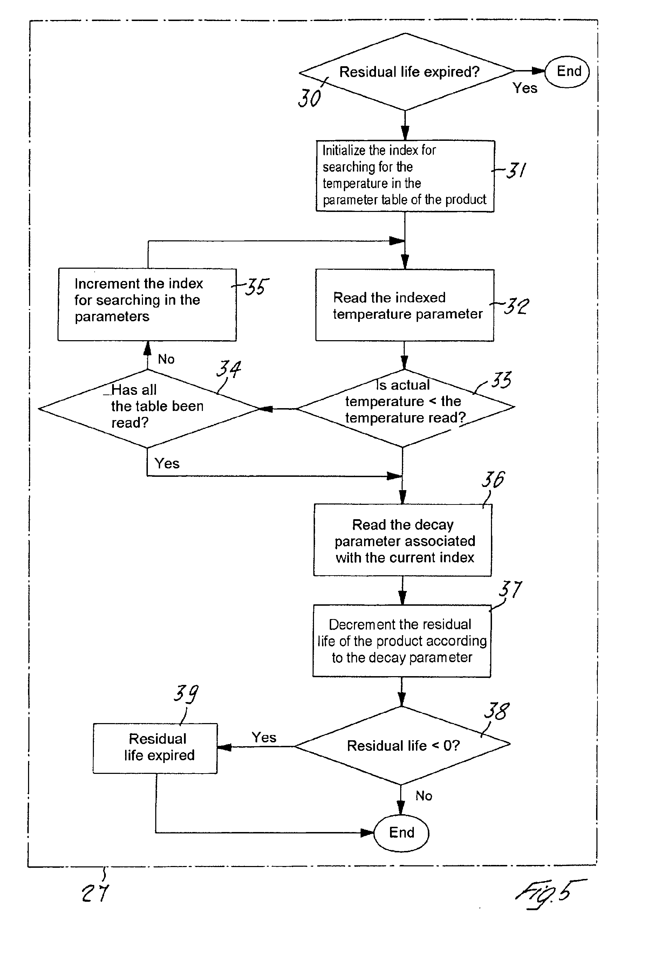

When the tests and parameter entry have been carried out on the device, the main operating cycle, as illustrated in Figure 4, commences; the data from the temperature sensor 2 are collected (steps 23 to 25 in the diagram) at relatively short time intervals, for example every 10 seconds (step 22). After a certain number of temperature readings, for example once every 60 seconds (26), the procedure of calculating the decay of the product (27) is started, using the curves of the type shown in Figure 2. The data relating to the residual life of the product are then updated (28).

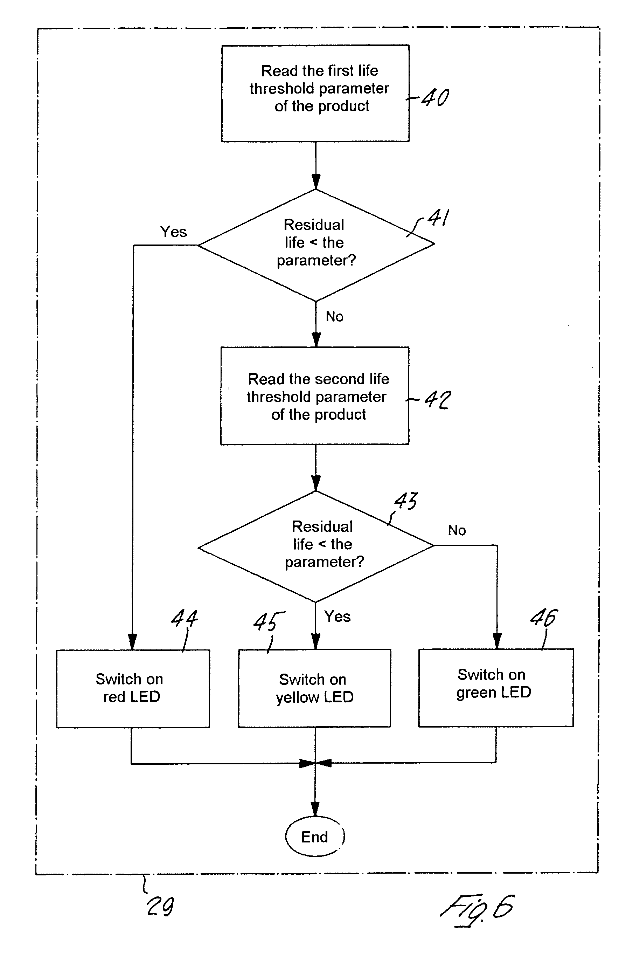

In particular, the procedure of calculating the decay of the product is carried out as shown in the flow diagram of Figure 5; the first step is to check whether the residual life of the product has expired. After this, the data on the temperature readings (31, 32) are entered, in order to check the decay of the properties (36) of the product associated with any variations of the temperature from that considered optimal for the preservation of the product (steps 33 to 35). The data relating to the residual life of the product are then updated (37) according to the changes which have occurred in the preservation history of the product, and finally another check is made as to whether the residual life of the product has expired (38, 39).

An example of the process carried out according to the method for evaluating the residual life of a product according to the present invention is shown in Figure 2 in the solid portion of line indicated by A. At the time to = 0, corresponding to the origin of the x and y axes, the product has 100% of its properties p which will become zero at the end of different time intervals t_{T1}, t_{T2}, t_{T3}, t_{Topt}, according to the preservation temperature T, where Tₒₚₜ is entered as the optimal preservation temperature of the product and Tₒₚₜ<T₁<T₂<T₃ (the various isotherms in the graph are identified by the corresponding temperatures). If, as shown in the portion A, the preservation temperature T varies during the period of preservation of the product, the properties of the product will be altered. In detail, the product is stored for a time t₁ at the temperature Tₒₚₜ, and then undergoes a temperature rise to T₁, at which temperature it is stored until the time t₂. After rapid heating which brings the product to T₂, the product is brought back to Tₒₚₜ. However, its residual life will be determined by the residual properties of the product, which are considerably diminished after the thermal changes to which the product has been subjected. In particular, its properties *p* will be equal to those still observable at the moment at which it was brought to T₂; the total preservation time of the product will therefore be t_{Topt} - Δt, where Δt is what is known as the "lost preservation time" and the residual life of the product is that which extends from t₃ to t_{Topt.}

The information on decay which has been obtained as described above is displayed, for example, by means of the LEDs 103, 203 and 303 shown in Figure 1. The flow diagram of Figure 4 indicates a procedure for controlling the said LEDs (29), which is started by pressing the button (step 21) which controls the switch 104; the procedure for controlling the LEDs is described in detail in Figure 6. When this procedure is started, the data obtained as described above are compared with the first life threshold parameter of the product (steps 40, 41) and then with the second threshold parameter of the product (42, 43). The illumination of the red LED (44) and the yellow LED (45) indicate that the residual life of the product has expired or is close to expiry; if this is not the case, the green LED (46) is illuminated. The introduction of a plurality of threshold parameters makes it possible to provide more a detailed indication of the state of preservation of the product, by adding further information (the illumination of the yellow LED) between that relating to the expired product and that relating to a product which is still in good condition.

Clearly, the choice of a light-emitting diode (LED) display can be considered as an alternative to other display means, which may be permanent, and which can display the whole preservation history of the product, possibly in schematic form.

As stated previously, the device according to the invention can be provided with a plurality of sensors for measuring different variables which affect the preservation of the product, and the effect of these variables on the product life will be considered in the processing of the residual life data.

Additionally, the processing unit can be implemented in its calculation capacity or in its data storage capacity, and can be provided with means for transferring the data obtained to other equipment.

The program used in the processing unit of the device according to the invention can be implemented by means of a wide range of compilation methods, but must in any case be capable of executing the steps of the method according to the present invention.

## Claims

1. Device for indicating the residual life of industrial products, comprising means (2) for sensing one or more environmental variables correlated with the preservation of the product, positioned on the interface between the product and the environment; processing means (1) for acquiring the data read by the sensor means (2), entering these data into a suitable program for evaluating the residual life of the product on the basis of these data, and returning the data relating to the residual life of the said product; and means (103, 203, 303) of displaying the data concerning the residual life of the product, controlled by the said processing unit (1).

2. Device according to Claim 1, **characterized in that** the said device comprises connection means (105, 205) for entering data into the said processing means (1).

3. Device according to Claim 1 or 2, in which the said display means comprise a plurality of optical indicators (103, 203, 303).

4. Device according to Claim 3, in which the said display means comprise three light-emitting diodes (103, 203, 303) having three different emission colours.

5. Device according to Claim 3 or 4, in which the said device comprises switch means (104) for starting the procedure for controlling the said optical indicators (103, 203, 303).

6. Method for evaluating the residual life of a product, comprising the following steps: entering the characteristic parameters of a single type of product into a set of generic functions which represent the decay of the properties of a product as a function of time, a constant value of at least one environmental variable being assigned to each function; measuring, instant by instant, the values relating to the said environmental variable; entering data relating to the said values into the set of functions; determining the preservation history of the product and its residual life on the basis of the entered data; and displaying the residual life data determined in this way.

7. Method according to Claim 6, in which the said characteristic parameters comprise the maximum life of the product in optimal preservation conditions and the law of the variation of its properties as a function of the variation of the said environmental variable.

8. Computer program, directly loadable into the internal store of an electronic computer, comprising the appropriate software code for executing the steps of the method according to Claim 6 or 7, when the said program is started in a computer.
